# EUROPEAN PATENT SPECIFICATION

(11) **EP 0 435 483 B1**
(45) Date of publication and mention of the grant of the patent: **26.10.1994**
(21) Application number: 90313142.3
(22) Date of filing: 04.12.1990
(51) Int. Cl.: A61K 7/06, A61K 7/48

(54) **Cosmetic composition**
Kosmetische Präparate
Composition cosmétique

(30) Priority: 07.12.1989 GB 8927703
(43) Date of publication of application: 03.07.1991
(73) Proprietor: UNILEVER PLC, London EC4P 4BQ (GB); UNILEVER N.V., 3013 AL Rotterdam (NL)
(72) Inventor: Pereira, Mavis Claire, Bebington, Wirral, Merseyside L63 3JW (GB); Thom, David, Bebington, Wirral, Merseyside L63 3JW (GB)
(74) Representative: Ford, Michael Frederick

(56) References cited:
- EP-A- 0 007 785
- EP-A- 0 271 925
- EP-A- 0 347 198
- EP-A- 0 373 661
- STN, FILE SUPPLIER KARLSRUHE, FILE CHEMICAL ABSTRACT, vol. 112, no. 14, 15th
- December 1989, abstract no. 124942j, Columbus, Ohio, US; & JP-A-1 236 936 (KOBAYASHI KASEI, CO., LTD) 21-09-1989 (Cat. X,Y) (Claims 1,3,6-10,13,15)

## Description

### FIELD OF INVENTION

The invention relates to a water-in-oil emulsion suitable for topical application to human skin and/or hair. More particularly, the invention is concerned with an exceptionally stable water-in-silicone oil emulsion comprising an inorganic electrolyte, which together with the silicone ingredients, and ideally a hydroxyalkanoic acid and an alkane diol provides a product having exceptional cosmetic and sensory properties aimed at improving the quality, particularly the plasticity of skin to which it is applied, either as a beauty aid or in the treatment of damaged or diseased skin.

### BACKGROUND TO INVENTION

A soft, supple and flexible skin has a marked cosmetic appeal and is an attribute of normal functioning epidermis.

As human skin ages with advancing years, the epidermis can become folded or ridged or furrowed to form wrinkles which signal the loss of youthful appearance and herald the transition to old age. This transition can occur prematurely with young people, especially those who expose themselves to excessive doses of sunlight. Also, the outer layer of the epidermis, that is the stratum corneum, can become dry and flaky following exposure to cold weather, or excessive contact with detergents or solvents which result in loss of skin moisture with the result that the skin loses its soft, supple and flexible characteristics.

Emollients such as fats, phospholipids and sterols have in the past been used to soften wrinkled or dry skin, but it is apparent that these emollients are only partially effective as a remedy for skin in poor condition.

### PRIOR ART

The use of 2-hydroxyalkanoic acids for enhancing the quality of human skin following topical application thereto has already been described.

Thus, EP-A 0 007 785 (Unilever) discloses skin treatment compositions incorporating α-hydroxycaproic acid or α-hydroxycaprylic or mixtures thereof, the compositions having a pH value of less than 7, usually from 2 to 4.

It is also proposed in US 4 105 782 (Yu & Van Scott) to use amines or ammonium salts of α-hydroxyacids in the treatment of acne or dandruff and, in the Yu & Van Scott patents US 4 105 783 and US 4 197 316, to use such compounds in the treatment of dry skin. US 4 234 599 (Yu & Van Scott) discloses the use of α-hydroxyacids, and their esters or amine salts in the treatment of keratoses. In US 4 363 815 (Yu & Van Scott) it is proposed to use
α-hydroxyacids or β-hydroxyacids or keto acids or their derivatives, in a composition for treating skin conditions.

According to GB 1 471 679 (Avon), it is known to use alkali metal salts of C₂ - C₅ α-hydroxycarboxylic acids in moisturising compositions.

In DE 2 110 993 (Henkel), there are disclosed alkali metal salts of C₄ - C₁₀ α-hydroxycarboxylic acids, and the sodium salt of α-hydroxycaprylic acid is mentioned.

EP-A-0271925 describes water-in-oil based cosmetic compositions for use as make-up, which comprise pigment which is rendered hydrophobic by a coating of polysiloxane. Various other ingredients of the compositions are mentioned, including silicones, surfactants such as particular silicone surfactants and various optional conventional ingredients such as humectants and electrolytes, among others.

EP-A-0373661 discloses stable water-in-silicone oil emulsion type cosmetics having a controlled viscosity and which include a specific mixture of emulsifiers, including a silicone surfactant and various salts.

JP-A-01236936 discloses water-in-oil cosmetic emulsion compositions containing sugars and/or inorganic salts, polyalcohols and polysiloxanes. Silicone surfactants are disclosed as possible ingredients of the compositions.

### SUMMARY OF INVENTION

It is apparent that some emulsions, such as classical oil-in-water emulsions, containing a hydroxyalkanoic acid, such as 2-hydroxyoctanoic acid, suffer from the disadvantage that they lack sufficient stability over long periods of storage at temperatures that may vary from below 0°C to up to 45°C, that is conditions to which such emulsions can be subjected following manufacture and prior to sale and use by the consumer. This is believed to be due at least partly to the solubilisation of the hydroxyalkanoic acid by the emulsifiers conventionally used in such products, with the consequence that separation of oil and water phases can ensue.

A further consequence of this solubilisation is to release the hydroxyalkanoic acid prematurely from the emulsion, so that its delivery to the skin when the emulsion is applied topically is less efficient and effective. The sensory properties of such emulsions can be poor, due to the presence of the hydroxyalkanoic acid in the continuous phase. This then exerts a dominating influence on the sensory profile, with the consequence that residual stickiness on the skin can be experienced.

Clearly, there is a need to provide emulsions of the type described which are not only stable at extremes of temperature over long periods of storage, but which also retain the ability to deliver to the skin in the most efficient and effective manner, an hydroxyalkanoic acid having exceptional skin benefit properties, while avoiding residual stickiness that can be experienced.

It has now surprisingly been found that certain 2-hydroxyalkanoic acids can be incorporated in an emulsion based on a polydimethylcyclosiloxane and a silicone emulsifier ingredient, together with an inorganic electrolyte, to provide an exceptionally stable emulsion, which has superior properties for delivery to the skin of the 2-hydroxyalkanoic acid, as well as having considerable consumer appeal by virtue of its superior skin feel.

In the absence of a 2-hydroxyalkanoic acid, the emulsion can provide a stable vehicle for the delivery to skin of other skin benefit materials.

Stability over a very long period of storage is further enhanced by incorporating a special diol. The emulsion can also provide a matrix which acts as a vehicle for other skin and/or hair benefit substances.

### DEFINITION OF THE INVENTION

Accordingly, the invention provides a water-in-silicone oil emulsion, suitable for topical application to mammalian skin or hair, which comprises, in addition to water;
i. from 1 to 50% by weight of a volatile polydimethylsiloxane,
ii. from 0.1 to 25% by weight of a silicone surfactant comprising a polymer of dimethyl polysiloxane with polyoxyethylene and/or polyoxypropylene side chains having a molecular weight of from 10,000 to 50,000 and having the structure: 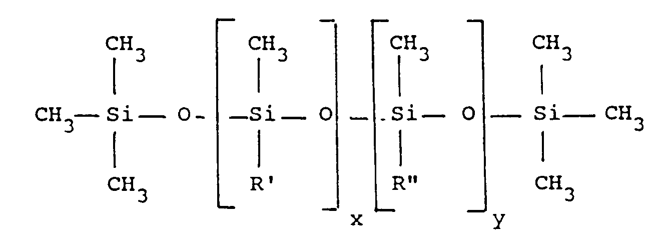 wherein the groups R' and R'' are each chosen from -H, C₁₋₁₈ alkyl and 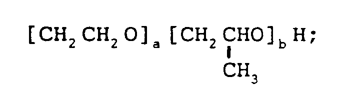 a has a value of from 9 to 115,
   b has a value of from 0 to 50,
   x has a value of from 133 to 673,
   y has a value of from 25 to 0.25;
iii. from 0.001 to 10% by weight of an inorganic electrolyte;
iv. from 1 to 50% by weight of an alkane diol having from 2 to 10 carbon atoms; and
v. from 0.1 to 10% by weight of a 2-hydroxyalkanoic acid having from 3 to 28 carbon atoms, or a salt, soap, acid-soap complex thereof, or a mixture thereof.

### DISCLOSURE OF THE INVENTION

The emulsion of the invention is a water-in-silicone oil emulsion, which is particularly suitable for topical application to mammalian skin or hair, especially that of the human subject. The emulsion is unusual in as much as it is exceptionally stable and retains superior sensory attributes, this being due to the careful choice of an inorganic electrolyte, together with a silicone emulsifier and volatile silicone. Stability during storage over a very long period of time of up to 3 years is further enhanced by incorporation of a an alkane diol, even in the presence of an hydroxyalkanoic acid

The emulsion can also provide a vehicle for other skin and/or hair benefit substances which can thereby be applied, with much greater ease and control to the skin or hair at an appropriate concentration suited to their intended benefit.

### The Polydimethylsiloxane

The emulsion of the invention comprises a volatile polydimethylsiloxane such as polydimethylcyclosiloxane having a viscosity of less than 5mm²s⁻¹, examples of which are DOW CORNING 344 Fluid (tetramer) and DOW CORNING 345 Fluid (pentamer), and volatile hexamethyldisiloxane having a viscosity of not more than 0.65mm²s⁻¹, for example DOW CORNING 200 Fluid (0.65mm²s⁻¹).

The preferred volatile siloxane is polydimethylcyclosiloxane (pentamer).

The emulsion will normally comprise from 1 to 50%, preferably from 5 to 20% by weight of the volatile siloxane.

### Silicone Surfactant

The emulsion of the invention also comprises a high molecular weight silicone surfactant which acts as an emulsifier.

The silicone surfactant is a high molecular weight polymer of dimethyl polysiloxane with polyoxyethylene and/or polyoxypropylene side chains having a molecular weight of from 10,000 to 50,000 and having the structure: 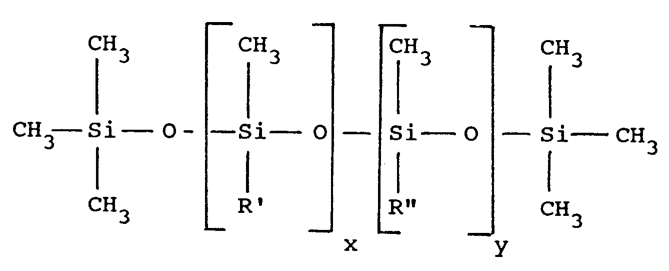
where the groups R' and R'' are each chosen from -H, C₁₋₁₈ alkyl and 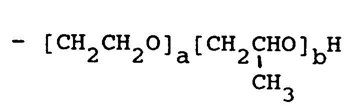
a has a value of from 9 to 115,
b has a value of from 0 to 50,
x has a value of from 133 to 673,
y has a value of from 25 to 0.25.

Preferably, the dimethyl polysiloxane polymer is one in which:
a has a value of from 10 to 114
b has a value of from 0 to 49
x has a value of from 388 to 402
y has a value of from 15 to 0.75
one of groups R' and R'' being lauryl, and the other having a molecular weight of from 1000 to 5000.

A particularly preferred dimethyl polysiloxane polymer is one in which:
a has the value 14
b has the value 13
x has the value 249
y has the value 1.25
The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include those given above.

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The emulsion according to the invention will normally comprise from 0.1 to 25%, preferably from 0.5 to 15% by weight of the silicone surfactant.

### Non-volatile siloxane

The emulsion can also, optionally, comprise a non-volatile siloxane such as a polydimethylsiloxane having a viscosity in excess of 5mm²s⁻¹, for example, from 50 to 1000mm²s⁻¹ for example DOW CORNING 200 Fluids (standard viscosities 50-1000mm²s⁻¹).

### The inorganic electrolyte

The emulsion of the invention also comprises an inorganic electrolyte which serves to improve the stability of the emulsion, particularly when subjected during storage to extremes of temperature.

Examples of inorganic electrolytes include salts, such as alkali metal and ammonium halides, sulphates, nitrates, carbonates and bicarbonates in either anhydrous or hydrated form.

Particularly preferred salts include sodium chloride, potassium chloride and ammonium chloride.

The emulsion according to the invention will normally comprise from 0.001 to 10%, preferably from 0.1 to 10%, more preferably from 0.2 to 5% by weight of the inorganic electrolyte.

### The hydroxyalkanoic acid

The emulsion of the invention also comprises a 2-hydroxyalkanoic acid having from 3 to 28 carbon atoms.

Examples of hydroxyalkanoic acids include:
2-hydroxypropanoic acid
2-hydroxyhexanoic acid
2-hydroxyoctanoic acid
2-hydroxydecanoic acid
2-hydroxydodecanoic acid
2-hydroxytetradecanoic acid
2-hydroxyhexadecanoic acid
2-hydroxyoctadecanoic acid
2-hydroxyeicosanoic acid
2-hydroxydocosanoic acid
2-hydroxytetracosanoic acid
2-hydroxyhexacosanoic acid, and
2-hydroxyoctacosanoic acid
Particularly preferred hydroxyalkanoic acids are those having from 3 to 8 carbon atoms, especially
2-hydroxypropanoic acid, and
2-hydroxyoctanoic acid.

The 2-hydroxyalkanoic acids can also be present in the emulsion in the form of their acid-soap complexes having from 6 to 56 carbon atoms, preferred examples of which have an elemental analysis of:

(Cₘ H_{2m-½} 0₃) (Cₙ H_{2n-½} 0₃) M

where m and n have the same or different values, and each is an integer of from 6 to 28, and M is a cation. The cation M is a monovalent ion such as potassium, sodium, ammonium or a substituted ammonium.

A particularly preferred example of the acid-soap complex is that derived from two molecules of 2-hydroxyoctanoic acid which has the empirical formula C₁₆H₃₁O₆Na, as disclosed in EP A O 347 198.

The 2-hydroxyalkanoic acids can also be present in the form of a salt or a soap thereof, or even as a mixture of any of the abovementioned forms in which the acid may be present.

The emulsion according to the invention will normally comprise from 0.1 to 10%, preferably from 0.5 to 5% by weight of the hydroxyalkanoic acid, its acid-soap complex, its salt or soap, or a mixture of any of these.

### The alkane diol

The emulsion of the invention also comprises an alkane diol, or a mixture thereof, which serves further to improve and prolong the stability of the emulsion, particularly when a very long period of storage, for example of at least 12 months or even up to 3 years, is anticipated.

The alkane diols for this purpose are those having from 2 to 10 carbon atoms in the molecule. Examples of particularly preferred alkane diols are:
ethane diol
propane-1,2-diol
propane-1,3-diol
butane-1,3-diol
butane-1,4-diol
butane-2,3-diol
pentane-1,5-diol
hexane-1,6-diol
octane-1,8-diol, and
decane-1,10-diol
An especially preferred alkane diol is butane-1,3-diol.

The emulsion according to the invention will preferably comprise from 1 to 50%, most preferably from 1 to 25% by weight of the alkane diol.

### OTHER INGREDIENTS

### Cosmetically Acceptable Vehicle

The emulsion of the invention can optionally comprise a cosmetically acceptable vehicle, in addition to water, to act as a dilutant, dispersant or carrier for other materials present in the emulsion, so as to facilitate their distribution when the emulsion is applied to the skin and/or hair.

Vehicles other than water can include liquid or solid emollients, solvents, humectants, thickeners and powders. Examples of each of these types of vehicle, which can be used singly or as mixtures of one or more vehicles, are as follows:
Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, di-n-butyl sebacate, isopropyl myristate, isopropyl palmitate, isopropyl stearate, butyl stearate, polyethylene glycol, lanolin, cocoa butter, corn oil, cotton seed oil, tallow, lard, olive oil, palm kernel oil, rapeseed oil, safflower seed oil, jojoba oil, evening primrose oil, soybean oil, sunflower seed oil, avocado oil, olive oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;
Propellants, such as propane, butane, isobutane, dimethyl ether, carbon dioxide, nitrous oxide;
Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate, titanium dioxide, titanium dioxide-coated mica.

The cosmetically acceptable vehicle, when present, will usually form up to to 99.9%, preferably from 10 to 99% by weight of the emulsion, and can, in the absence of other cosmetic adjuncts, form the balance of the emulsion.

### Cosmetic Adjuncts

Examples of conventional adjuncts which can optionally be employed include preservatives, such as para-hydroxy benzoate esters; antioxidants, such as butyl hydroxy toluene; solvents, such as ethyl alcohol and isopropanol; humectants, such as glycerol, sorbitol, 2-pyrrolidone-5-carboxylate, dibutylphthalate, gelatin, polyethylene glycol, preferably PEG 200-600; buffers, such as lactic acid together with a base such as triethanolamine or sodium hydroxide; waxes, such as beeswax, ozokerite wax, paraffin wax; plant extracts, such as Aloe vera, cornflower, witch hazel, elderflower, cucumber; thickeners; activity enhancers; colourants; perfumes; and sunscreens, such as p-aminobenzoic acid, ethylhexyl p-methoxycinnamate and 2-ethoxyethyl-p-methoxycinnamate.

Cosmetic adjuncts can form up to 50% by weight of the emulsion and can conveniently form the balance of the emulsion.

### pH

The aqueous phase of the emulsion according to the invention should preferably have a pH value of from 3.5 to <7.

### Process for preparing the emulsion

The invention also provides a process for the preparation of an emulsion for topical application to skin and/or hair which comprises the step of incorporating into the emulsion a volatile polydimethylsiloxane, a silicone surfactant, an inorganic electrolyte, a 2-hydroxyalkanoic acid and an alkane diol, as herein defined.

### Use of the emulsion

The emulsion according to the invention is intended primarily as a product for topical application to human skin, particularly dry skin, when repeated application can alleviate the dry condition, and restore the skin to a more natural, soft, supple, healthy state. The emulsion can also be used to treat the hair and the scalp.

In use, a small quantity of the emulsion, for example from 1 to 5 ml, is applied to the affected area of skin or hair, from a suitable container or applicator and, if necessary, it is then spread over and/or rubbed into the skin or hair using the hand or fingers or a suitable device.

### PRODUCT FORM AND PACKAGING

The topical skin and/or hair treatment emulsion of the invention can be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. The emulsion can be packaged in a suitable container to suit its viscosity and intended use by the consumer. For example a lotion or a fluid cream can be packaged in a bottle or a roll-ball applicator or a propellant-driven aerosol device or a container fitted with a pump suitable for finger operation. When the emulsion is a cream, it can simply be stored in a non-deformable bottle or squeeze container, such as a tube or a lidded jar.

The invention accordingly also provides a closed container containing a cosmetically acceptable emulsion as herein defined.

### Evidence to demonstrate improvement in storage stability of the emulsion of the invention due to the presence of an alkane diol.

As has been stated herein before, stable emulsions can be obtained by incorporating certain 2-hydroxyalkanoic acids into an emulsion based on a polydimethylcyclosiloxane and a silicone emulsifier, together with an inorganic electrolyte and alkane diol. Such emulsions are satisfactory for use, provided they are not stored for an extended period of, say, more than 3 months, at an elevated temperature of 40°C or above. Accordingly, it is apparent that these emulsions which are subjected to long storage at high temperatures can suffer from partial separation of the water and oily phases.

When extended storage at high temperature is likely to be the case, then stability of the emulsion can be further enhanced by the incorporation of an alkane diol.

The evidence for improved stability of the emulsion according to the invention, due solely to the incorporation of butane-1,3-diol, as the alkane diol is given in the following experiment.

### Control formulation (without butane-1,3-diol)

A lotion in accordance with the invention but without butane-1,3-diol as the alkane diol, had the following formulation:

| Ingredient | Wt% |
|---|---|
| volatile siloxane (DC 345 Fluid) | 7.00 |
| silicone surfactant (DC 3225C) | 10.00 |
| isopropyl myristate | 7.00 |
| titanium dioxide | 0.15 |
| sodium chloride | 2.00 |
| 2-hydroxyoctanoic acid | 1.00 |
| 2-hydroxypropanoic acid | 5.00 |
| neutralising agent | q.s. |
| preservative | q.s. |
| perfume | q.s. |
| water | to 100% |

### Viscosity data

The viscosity of the above emulsion measured on the Brookfield (TC, 10rpm) was 15,000 mPas.

### Storage data

This control lotion showed 'thinning' (i.e., a drop in viscosity) after three months storage at 35°C and 45°C, and completed only two freeze/thaw cycles (-22°C) before syneresis set in, instead of the normal four cycles. Generally, a drop in viscosity is indicative of long-term instability of an emulsion.

### Test formulation (with added butane-1,3-diol)

A similar lotion to the control described above was prepared but with butane-1,3-diol added at different levels. These test lotions had the following formulations:

| Ingredient | Wt % | | |
|---|---|---|---|
| | A | B | C |
| volatile siloxane (DC 345) | 7.00 | 7.00 | 7.00 |
| silicone surfactant (DC 3225C) | 10.00 | 10.00 | 10.00 |
| isopropyl myristate | 7.00 | 7.00 | 7.00 |
| titanium dioxide | 0.15 | 0.15 | 0.15 |
| 2-hydroxyoctanoic acid | 1.00 | 1.00 | 1.00 |
| 2-hydroxypropanoic acid | 5.00 | 5.00 | 5.00 |
| sodium choloride | 2.00 | 2.00 | 2.00 |
| butane-1,3-diol | 5.00 | 7.50 | 10.00 |
| neutralising agent (triethanolamine) | q.s. | q.s. | q.s. |
| preservative | q.s. | q.s. | q.s. |
| perfume | q.s. | q.s. | q.s. |
| water | <-----to 100%-----> | | |

### Viscosity data

Viscosities measured on Brookfield (TB,10 rpm) immediately after manufacture were as follows:-

| Test formulation | Viscosity (mPas) |
|---|---|
| A | 10,500 |
| B | 8,360 |
| C | 17,600 |

### Storage data

Test formulations A, B and C each successfully completed four freeze/thaw cycles at -22°C and six months storage at 45°C (three months storage at this temperature is the industry standard minimum requirement) and at 35°C. No changes in viscosity were observed during storage testing.

### Conclusion

It was concluded from this comparative storage experiment that butane-1,3-diol dramatically enhanced the stability of the emulsion according to the invention when stored at a high temperature for an extended period.

### Examples

The invention is further illustrated by the following examples.

### Example 1

This example illustrates a lotion according to the invention.

| Ingredients | % w/w |
|---|---|
| silicone surfactant (DC 3225C) | 10.00 |
| volatile siloxane (DC 345 Fluid) | 14.00 |
| titanium dioxide | 0.20 |
| 2-hydroxyoctanoic acid | 1.00 |
| 2-hydroxypropanoic acid | 5.00 |
| butane-1,3-diol | 10.00 |
| sodium chloride | 2.00 |
| Neutralising agent (aqueous phase to pH 4.5) | q.s. |
| preservative | q.s. |
| perfume | q.s. |
| water | to 100 |

### Viscosity data

The viscosity of the above lotion measured on the Brookfield (TB, 10rpm) immediately after manufacture, was 5440 mPas. No change in viscosity was observed after 24 hrs.

### Storage data

The lotion was subjected to four Freeze/Thaw cycles between +20°C and -22°C, and no syneresis or deteriorative changes were observed. Subsequently the emulsion remained stable for at least six months at storage temperatures of 45°C, 35°C and 20°C.

### Example 2

This example illustrates a fluid cream according to the invention.

| Ingredients | % w/w |
|---|---|
| volatile siloxane (DC 345 Fluid) | 14.00 |
| silicone surfactant (DC 3225C) | 10.00 |
| sunscreen (octyl methoxycinnamate) | 1.00 |
| sunscreen (butyl methoxydibenzoylmethane) | 0.50 |
| titanium dioxide | 0.20 |
| butane-1,3-diol | 10.00 |
| sodium chloride | 2.00 |
| amino acid | 0.10 |
| 2-hydroxyoctanoic acid | 1.00 |
| 2-hydroxypropanoic acid | 5.00 |
| neutralising agent (aqueous phase to pH 4.5) | q.s. |
| preservative | q.s. |
| perfume | q.s. |
| water | to 100 |

### Viscosity data

The viscosity of the above fluid cream measured on the Brookfield (TB,10rpm) 24 hr after manufacture was 19,800 mPas.

### Storage data

The fluid cream was subjected to four Freeze/Thaw cycles between +20°C and -22°C, and no syneresis or deteriorative changes were observed. The emulsion will remained stable for a period of six months at storage temperatures of 45°C, 35°C and 20°C.

### Example 3

This example illustrates a cream according to the invention.

| Ingredients | % w/w |
|---|---|
| volatile siloxane (DC 345 Fluid) | 8.2 |
| silicone surfactant (DC 3225C) | 12.0 |
| mineral oil | 1.5 |
| petroleum jelly | 0.5 |
| titanium dioxide | 0.2 |
| sodium chloride | 2.0 |
| butane-1,3-diol | 13.5 |
| 2-hydroxyoctanoic acid | 1.0 |
| 2-hydroxypropanoic acid | 5.0 |
| neutralising agent (aqueous phase to 4.5) | q.s. |
| preservative | q.s. |
| water | to 100 |

### Viscosity data

The viscosity of the above cream measured on the Brookfield (TC,10rpm) 24 hr after manufacture was 50,000 mPas.

### Storage data

The cream was subjected to four Freeze/Thaw cycles between +20°C and -22°C, and no syneresis or deteriorative changes were observed. Subsequently the emulsion remained stable for at least fifteen months at storage temperatures of 45°C, 35°C and 20°C.

## Claims

1. A water-in-silicone oil emulsion, suitable for topical application to mammalian skin or hair, which comprises, in addition to water:
i. from 1 to 50% by weight of a volatile polydimethylsiloxane,
ii. from 0.1 to 25% by weight of a silicone surfactant comprising a polymer of dimethyl polysiloxane with polyoxyethylene and/or polyoxypropylene side chains having a molecular weight of from 10,000 to 50,000 and having the structure: 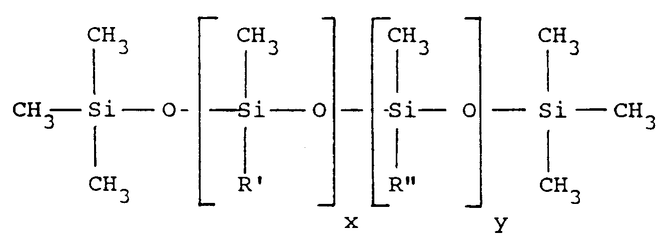 wherein the groups R' and R'' are each chosen from -H, C₁₋₁₈ alkyl and 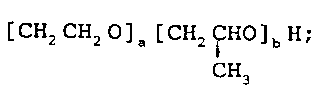 a has a value of from 9 to 115,
b has a value of from 0 to 50,
x has a value of from 133 to 673,
y has a value of from 25 to 0.25;
iii. from 0.001 to 10% by weight of an inorganic electrolyte;
iv. from 1 to 50% by weight of an alkane diol having from 2 to 10 carbon atoms; and
v. from 0.1 to 10% by weight of a 2-hydroxyalkanoic acid having from 3 to 28 carbon atoms, or a salt, soap, acid-soap complex thereof, or a mixture thereof.

2. An emulsion according to claim 1, in which the volatile polydimethylsiloxane is polydimethylcyclosiloxane (tetramer).

3. An emulsion according to claim 1, in which the volatile polydimethylsiloxane is polydimethylcyclosiloxane (pentamer).

4. An emulsion according to any preceding claim, in which the silicone surfactant is one in which:
a has a value of from 10 to 114
b has a value of from 0 to 49
x has a value of from 388 to 402 and
y has a value of from 15 to 0.75;
one of groups R' and R'' being lauryl, and the other having a molecular weight of from 1000 to 5000.

5. An emulsion according to any preceding claim, in which the silicone surfactant is one in which:
a has the value 14
b has the value 13
x has the value 249, and
y has the value 1.25.

6. An emulsion according to any preceding claim, in which the inorganic electrolyte is chosen from alkali metal and ammonium halides, sulphates, carbonates and mixtures thereof.

7. An emulsion according to any preceding claim, in which the inorganic electrolyte is sodium chloride.

8. An emulsion according to any preceding claim, in which the alkane diol is chosen from:
propane-1,2-diol
propane-1,3-diol
butane-1,3-diol
butane-1,4-diol
butane-2,3-diol
and mixtures thereof.

9. An emulsion according to any preceding claim in which the 2-hydroxyalkanoic acid is chosen from 2-hydroxypropanoic acid, 2-hydroxyoctanoic acid and mixtures thereof.

10. An emulsion according to any preceding claim, in which the 2-hydroxyalkanoic acid is in the form of an acid-soap complex having the structure:
(CₘH_{2m-½}O₃) (Cₙ H_{2n-½}O₃) M
where m and n have the same or different values, and each is an integer of from 6 to 28, and where M is a monovalent ion.

11. An emulsion according to claim 10, in which the acid-soap complex has the empirical formula:
C₁₆H₃₁O₆Na.

12. An emulsion according to any preceding claim, which further comprises a non-volatile siloxane.

13. A process for preparing an emulsion according to any preceding claim, which comprises the step of emulsifying a volatile polydimethylsiloxane, a silicone surfactant, an inorganic electrolyte, an alkane diol and a 2-hydroxyalkanoic acid.

14. The use of an emulsion as claimed in any one of claims 1 to 12 in alleviating skin dryness following topical application of the emulsion to dry skin.

## Patentansprüche

1. Wasser-in-Siliconöl-Emulsion, geeignet zur örtlichen Anwendung auf Haut oder Haar von Säugern, umfassend zusätzlich zu Wasser;
i. 1 bis 50 Gew.-% eines flüchtigen Polydimethylsiloxans;
ii. 0,1 bis 25 Gew.-% eines Silicontensids, umfassend ein Polymer von Dimethylpolysiloxan mit Polyoxyethylen und/oder Polyoxypropylenseitenketten, ein Molekulargewicht von 10 000 bis 50 000 und die Struktur aufweisend: 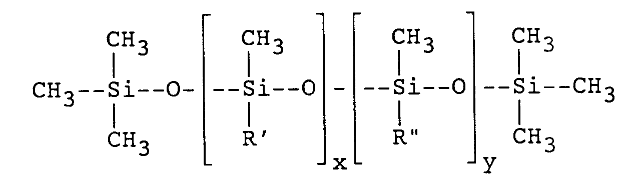 worin die Gruppen R' und R'' jeweils ausgewählt sind aus -H, C₁₋₁₈-Alkyl und 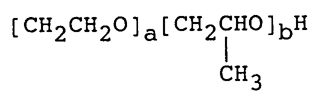 a einen Wert von 9 bis 115 aufweist,
b einen Wert von 0 bis 50 aufweist,
x einen Wert von 133 bis 673 aufweist, und
y einen Wert von 25 bis 0,25 aufweist;
iii. 0,001 bis 10 Gew.-% eines anorganischen Elektrolyten;
iv. 1 bis 50 Gew.-% eines Alkandiols mit 2 bis 10 Kohlenstoffatomen; und
v. 0,1 bis 10 Gew.-% einer 2-Hydroxyalkansäure mit 3 bis 28 Kohlenstoffatomen oder einem Salz, einer Seife, eines Säure-Seife-Komplexes davon oder eines Gemisches davon.

2. Emulsion nach Anspruch 1, wobei das flüchtige Polydimethylsiloxan Polydimethylcyclosiloxan (Tetramer) ist.

3. Emulsion nach Anspruch 1, wobei das flüchtige Polydimethylsiloxan Polydimethylcyclosiloxan (Pentamer) ist.

4. Emulsion nach einem vorangehenden Anspruch, wobei in dem Silicontensid:
a einen Wert von 10 bis 114 aufweist,
b einen Wert von 0 bis 49 aufweist,
x einen Wert von 388 bis 402 aufweist, und
y einen Wert von 15 bis 0,75 aufweist;
eine der Gruppen R' und R'' Lauryl bedeutet und die andere ein Molekulargewicht von 1000 bis 5000 aufweist.

5. Emulsion nach einem vorangehenden Anspruch, wobei in dem Silicontensid:
a einen Wert von 14 aufweist,
b einen Wert von 13 aufweist,
x einen Wert von 249 aufweist, und
y einen Wert von 1,25 aufweist.

6. Emulsion nach einem vorangehenden Anspruch, wobei der anorganische Elektrolyt ausgewählt ist aus Alkalimetall- und Ammoniumhalogeniden, Sulfaten, Carbonaten und Gemischen davon.

7. Emulsion nach einem vorangehenden Anspruch, wobei der anorganische Elektrolyt Natriumchlorid ist.

8. Emulsion nach einem vorangehenden Anspruch, wobei das Alkandiol ausgewählt ist aus
Propan-1,2-diol
Propan-1,3-diol
Butan-1,3-diol
Butan-1,4-diol
Butan-2,3-diol
und Gemischen davon.

9. Emulsion nach einem vorangehenden Anspruch, wobei die 2-Hydroxyalkansäure ausgewählt ist aus 2-Hydroxypropansäure, 2-Hydroxyoctansäure und Gemischen davon.

10. Emulsion nach einem vorangehenden Anspruch, wobei die 2-Hydroxyalkansäure in Form eines Säure-Seife-Komplexes der Struktur:
(CₘH_{2m-½}O₃) (CₙH_{2n-½}O₃) M
vorliegt, wobei m und n die gleichen oder verschiedene Werte aufweisen und jeweils eine ganze Zahl von 6 bis 28 darstellen, und worin M ein einwertiges Ion ist.

11. Emulsion nach Anspruch 10, wobei der Säure-Seife-Komplex die empirische Formel:
C₁₆H₃₁O₆Na
aufweist.

12. Emulsion nach einem vorangehenden Anspruch, die zusätzlich nichtflüchtiges Siloxan umfaßt.

13. Verfahren zur Herstellung einer Emulsion nach einem vorangehenden Anspruch, umfassend den Schritt des Emulgierens eines flüchtigen Polydimethylsiloxans, eines Silicontensids, eines anorganischen Elektrolyten, eines Alkandiols und einer 2-Hydroxyalkansäure.

14. Verwendung einer Emulsion nach einem der Ansprüche 1 bis 12, bei der Milderung von Hauttrockenheit nach örtlicher Anwendung der Emulsion auf trockene Haut auftritt.

## Revendications

1. Emulsion d'eau dans huile siliconique qui convient pour des applications locales à la peau ou aux cheveux des mammifères, qui comprend en plus de l'eau :
i. de 1 à 50% en poids d'un polydiméthylsiloxane volatil,
ii. de 0,1 à 25% en poids d'un tensioactif siliconique comprenant un polymère de diméthylpolysiloxane avec des chaînes latérales de polyoxyéthylène et/ou polyoxypropylène ayant une masse moléculaire comprise entre 10000 et 50000 et répondant à la formule : 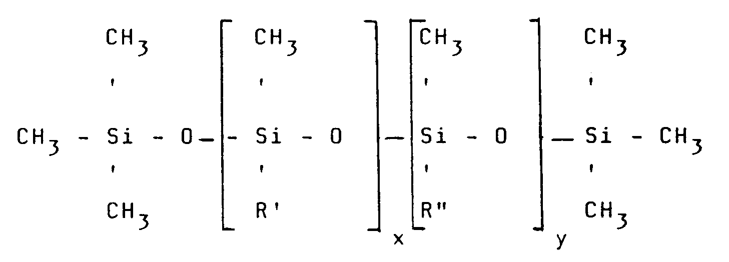 dans laquelle les groupes R' et R'' représentent chacun -H, alkyle en C₁₋₁₈ ou 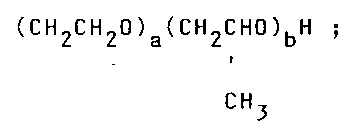 a est un nombre de 9 à 115,
b est un nombre de 0 à 50,
x est un nombre de 133 à 673,
y est un nombre de 25 à 0,25 ;
iii. de 0,001 à 10% en poids d'un électrolyte mineral:
iv. de 1 à 50% en poids d'un alcanediol contenant de 2 à 10 atomes de carbone ; et
v. de 0,1 à 10% en poids d'un acide 2-hydroxyalcanoïque contenant de 3 à 28 atomes de carbone ou d'un sel, savon, complexe acide-savon de celui-ci ou un mélange de ces derniers.

2. Emulsion selon la revendication 1, dans laquelle le polydiméthylsiloxane volatil est le polydiméthylcyclosiloxane (tetramère).

3. Emulsion selon la revendication 1, dans laquelle le polydiméthylsiloxane volatil est le polydiméthylcyclosiloxane (pentamère).

4. Emulsion selon l'une quelconque des revendications précédentes, dans laquelle le tensioactif siliconique est un produit dans lequel :
a est un nombre de 10 à 114,
b est nombre de 0 à 49,
x est un nombre de 388 à 402, et
y est un nombre de 15 à 0,75 ;
l'un des radicaux R' et R'' étant le radical lauryle et l'autre ayant une masse moléculaire de 1000 à 5000.

5. Emulsion selon l'une quelconque des revendications précédentes, dans laquelle le tensioactif siliconique est un produit dans lequel :
a est 14,
b est 13,
x est 249, et
y est 1,25.

6. Emulsion selon l'une quelconque des revendications précédentes, dans laquelle l'électrolyte minéral est choisi parmi les halogénures, sulfates, carbonates et leurs mélanges de métaux alcalins et d'ammonium.

7. Emulsion selon l'une quelconque des revendications précédentes, dans laquelle l'électrolyte minéral est le chlorure de sodium.

8. Emulsion selon l'une quelconque des revendications précédentes, dans laquelle l'alcanediol est choisi parmi :
le propane-1,2-diol,
le propane-1,3-diol,
le butane 2,3-diol,
le butane-1,4-diol, et
le butane 2,3-diol
et leurs mélanges.

9. Emulsion selon l'une quelconque des revendications précédentes, dans laquelle l'acide 2-hydroxyalcanoïque est choisi parmi l'acide 2-hydroxypropanoïque, l'acide 2-hydroxyoctanoïque et leurs mélanges.

10. Emulsion selon l'une quelconque des revendications précédentes, dans laquelle l'acide 2-hydroxyalcanoïque est sous forme d'un complexe acide-savon de formule :
(Cₘ H_{2m-½}O₃) (CₙH_{2n-½}O₃) M
dans laquelle m et n qui peuvent être identiques ou différents, représentent chacun un nombre entier de 6 à 28 et M est un ion monovalent.

11. Emulsion selon la revendication 10, dans laquelle le complexe acide-savon répond à la formule empirique :
C₁₆H₃₁O₆Na.

12. Emulsion selon l'une quelconque des revendications précédentes, qui comprend en outre un siloxane non volatil.

13. Procédé de préparation d'une émulsion selon l'une quelconque des revendications précédentes, qui consiste à émulsifier un polydiméthylsiloxane volatil, un tensioactif siliconique, un électrolyte minéral, un alcanediol et un acide 2-hydroxyalcanoïque.

14. Utilisation d'une émulsion selon l'une quelconque ds revendications 1 à 12 pour réduire la sécheresse de la peau par application de l'émulsion à la peau sèche.
